(19) Europäisches Patentamt
European Patent Office
Office européen des brevets

(11) Veröffentlichungsnummer: **0 185 679 B1**

(12) # EUROPÄISCHE PATENTSCHRIFT veröffentlicht nach Art. 158 Abs. 3 EPÜ

(45) Veröffentlichungstag der Patentschrift: **28.08.91**

(51) Int. Cl.5: **C07D 501/06**, C07D 417/12

(21) Anmeldenummer: **85902014.1**

(22) Anmeldetag: **04.04.85**

(86) Internationale Anmeldenummer: **PCT/EP85/00156**

(87) Internationale Veröffentlichungsnummer: **WO 85/04659 (24.10.85 85/23)**

Teilanmeldung 88116570 eingereicht am 06.10.88.

(54) **CEPHALOSPORINZWISCHENPRODUKTE, VERFAHREN ZU IHRER HERSTELLUNG UND IHRE VERWENDUNG.**

(30) Priorität: **10.04.84 AT 1197/84**
**10.04.84 AT 1198/84**

(43) Veröffentlichungstag der Anmeldung: **02.07.86 Patentblatt 86/27**

(45) Bekanntmachung des Hinweises auf die Patenterteilung: **28.08.91 Patentblatt 91/35**

(84) Benannte Vertragsstaaten: **BE CH DE FR GB IT LI**

(56) Entgegenhaltungen:
**EP-A- 0 037 380**
**DE-A- 3 433 147**

(73) Patentinhaber: **BIOCHEMIE Gesellschaft m.b.H.**
**A-6250 Kundl Tirol(AT)**

(72) Erfinder: **ASCHER, Gerd**
**A-6250 Kundl(AT)**
Erfinder: **STURM, Herbert**
**Urichstrasse 102**
**A-6500 Landeck(AT)**
Erfinder: **THALER, Heinrich**
**A-6322 Kirchbich 195(AT)**
Erfinder: **VEITH, Werner**
**Kaiserjägerstrasse 27**
**A-6330 Kufstein(AT)**

(74) Vertreter: **Kleine-Deters, Johannes et al**
**Sandoz AG Patentabteilung**
**CH-4002 Basel(CH)**

EP 0 185 679 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Rank Xerox (UK) Business Services

**Beschreibung**

Die Erfindung betrifft ein Verfahren zur Herstellung von Cephalosporinzwischenprodukten der Formel

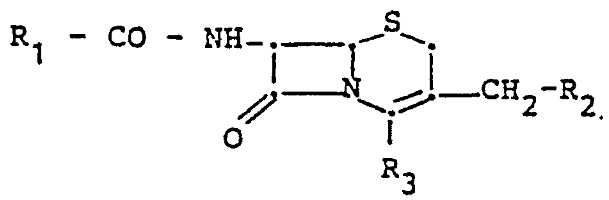

I

worin $R_1$ für die Gruppe der Formel

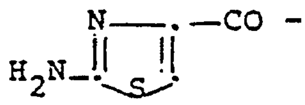

IIa

steht, $R_2$ für Wasserstoff, die Acetoxy-, die Carbamoyloxy-, eine S-Y-Gruppe, wobei Y einen unsubstituierten oder substituierten Heterocyclus bedeutet, oder $R_2$ für ein gegebenenfalls substituiertes Pyridinium der Formel

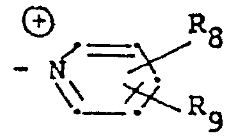

III

wobei $R_8$ und $R_9$ gleich oder verschieden sind und jeweils für Wasserstoff, Halogen, Alkyl, Hydroxy, Carboxamido, Alkoxycarbonyl, Amino, Monoalkylamino oder Dialkylamino stehen oder zusammen einen gegebenenfalls substituierten, 5- oder 6-gliedrigen carbocyclischen Ring bedeuten, steht und $R_3$ die Carboxyl-, die Carboxylat- oder eine Carbonsäureestergruppe bedeutet, und ihre Verwendung.

Aus den Cephalosporinzwischenprodukten der Formel I können wertvolle Cephalosporinantibiotika hergestellt werden.

In diesen Verbindungen kann $R_2$ Wasserstoff sein. Es kann auch Carbamoyloxy sein. Vorzugsweise bedeutet es aber Acetoxy, -S-Y oder gegebenen falls substituiertes Pyridinium. Geeignete Heterocyclen als Y sind bekannt. Bevorzugte Heterocyclen sind beispielsweise Thiadiazolyl, Diazolyl, Triazolyl, Tetrazolyl, Thiazolyl, Thiatriazolyl, Oxazolyl, Oxadiazolyl, Triazolylpyridyl, Purinyl, Pyridyl, Pyrimidinyl, Pyridazinyl, Pyrazolyl und Triazinyl. Diese Heterocyclen können unsubstituiert oder beispielsweise bis dreifach substituiert sein. Als Substituenten sind $(C_{1-4})$Alkyl,$(C_{1-4})$Alkoxy, Halogen, Trihalo-$(C_{1-4})$alkyl, Hydroxy, Oxo, Mercapto, Amino, Carboxyl, Carbamoyl, Di-$(C_{1-4})$alkylamino, Carboxymethyl, Carbamoylmethyl, Sulfomethyl und Methoxycarbonylamino geeignet. In der Literatur bisher als bevorzugt beschriebene Heterocyclen beinhalten Tetrazolyl, insbesondere 1-Methyl-1H-tetrazol-5-yl, und Triazinyl, insbesondere 1,2,5,6-Tetrahydro-2-methyl-5,6-dioxo-as-triazin-3-yl, 2,5-Dihydro-6-hydroxy-2-methyl-5-oxo-as-tiazin-3-yl oder 1,4,5,6,-Tetrahydro-4-methyl-5,6-dioxo-as-triazin-3-yl. Vorzugsweise ist $R_2$ Acetoxy, 1-Methyl-1H-tetrazol-5-ylthio, 2,5-Dihydro-6-hydroxy-2-methyl-5-oxo-as-triazin-3-ylthio oder Pyridinium.

Wie auf dem Gebiet der Cephalosporine bekannt, können die Verbindungen in Form der freien Säuren ($R_3$ = COOH) oder von Salzen, beispielsweise von Alkali- oder Erdalkalimetallsalzen, vorzugsweise von Alkalimetallsalzen, wie Natriumsalzen, vorliegen. Weiters können die Verbindungen auch in Form von Estern, beispielsweise in Form des Pivaloyloxymethylesters vorliegen, aber auch als andere Ester, wie beispielsweise Acetoxymethyl-, 1-Acetoxyethyl-, 1-Ethoxycarbonyloxyethyl-, 5-Indanoyl- oder vorzugsweise Hexanoylmethyl-, Phthalidyl-, Carbethoxymethoxymethyl- oder 3-Carbethoxy-1-acetonylester. Steht $R_2$ für eine gegebenenfalls substituierte Pyridiniumgruppe, so können die Verbindungen der Formel I auch als innere Salze vorliegen. Weitere $R_2$-Gruppen, die basische Reste enthalten, können ebenfalls in Form von Salzen vorliegen.

Wie bereits erwähnt, sind die aus den Verbindungen der Formel I herstellbaren Cephalosporinantibiotika bekannt, und verschiedene Methoden zu ihrer Herstellung wurden bereits vorgeschlagen. Eine dieser

Methoden beinhaltet die Acylierung des entsprechenden, gegebenenfalls geschützten 7-Aminocephalosporansäurederivats mit einem aktiven Derivat einer Säure der Formel

$R_1$ - COOH A

worin $R_1$ obige Bedeutung besitzt.

Die verschiedenen vorgeschlagenen aktiven Derivate beinhalten auch aktive Ester.

Eine bei der Herstellung der Verbindungen der Formeln I entstehende Schwierigkeit besteht darin, daß in der Praxis der Aminosubstituent im Thiazolylring der Seitenkette vor der Aktivierung der Säurefunktion geschützt werden muß, da sonst kompetitive Reaktionen zu starken Ausbeuteverminderungen an Endprodukt führen können. Die Einführung von Schutzgruppen vor dem Acylierungsschritt und ihre spätere Abspaltung verursacht aber meist verminderte Ausbeuten und Reinheit des Endproduk- tes und erhöht wesentlich Reaktionszeit, Energie, Aufwand und Kosten.

Die vorliegende Erfindung umfaßt ein Verfahren zur Herstellung der gewünschten Verbindungen der Formel I in hoher Ausbeute und Reinheit, ohne daß der Aminosubstituent im Thiazolylring der Seitenkette geschützt werden muß.

Insbesondere umfaßt die vorliegende Erfindung ein Verfahren zur Herstellung von Verbindungen der Formel I und ihrer Salze, dadurch gekennzeichnet, daß man eine Verbindung der Formel

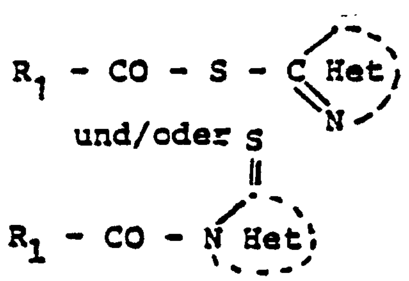

IV

und/oder

IVa

worin $R_1$ obige Bedeutung besitzt und

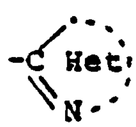 bzw. 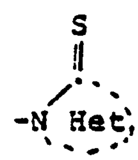

für einen 5- oder 6-gliedrigen Heterocyclus, der zusätzlich zum N-Atom ein oder zwei weitere Heteroatome beinhalten kann, beispielsweise O, N und S, und der substituiert oder mit einem gegebenenfalls substituierten Benzolring annelliert sein kann, stehen, mit einer Verbindung der Formel

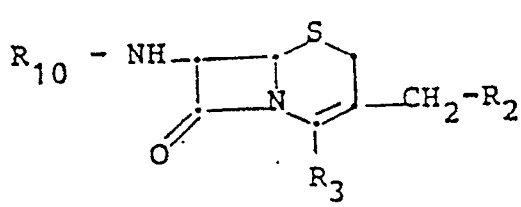

V

worin $R_2$ und $R_3$ obige Bedeutung besitzen und $R_{10}$ für Wasserstoff oder eine Aminoschutzgruppe steht, umsetzt und gewünschtenfalls ein erhaltenes Endprodukt entschützt und gegebenenfalls ein erhaltenes Endprodukt, worin $R_3$ für COOH steht, in ein Salz überführt oder umgekehrt.

Die Verbindungen der Formel IV können auch in Form der Formel IVa vorliegen, wobei das Verhältnis der beiden Formen zueinander von den Reaktionspartnern und den Reaktionsbedingungen abhängig ist. Wenn Verbindungen der Struktur der Formel IV angeführt werden, so umfassen diese immer auch Verbindungen der Struktur der Formel IVa.

Das erfindungsgemäße Verfahren wird vorzugsweise in einem inerten Lösungsmittel oder in einem Gemisch eines solchen Lösungsmittels mit Wasser, z.B in einem chlorierten Kohlenwasserstoff, wie

Dichlormethan, oder in einem Säureester, wie Essigsäureethylester, oder in Azeton, Dimethylformamid oder Dimethylsulfoxid bei einer Temperatur von -40 bis +60° C, insbesondere bei -15 bis +25° C und vorteilhafterweise bei 0 bis 20° C ausgeführt. Die Reaktionsdauer beträgt etwa 1/2 bis 48 Stunden. Die Reaktanden der Formel IV und V werden entweder in stöchiometrischen Mengen eingesetzt oder es wird ein Überschuß von bis zu 25 % an Verbindung der Formel IV verwendet.

Bei der Herstellung von Verbindungen der Formel I, worin $R_3$ COOH bedeutet, wie auch ihrer Salze kann die Carboxylgruppe im Ausgangsprodukt der Formel V geschützt sein. Geeignete Schutzgruppen sind bekannt und umfassen nicht nur die oben angeführten Bedeutungen, sondern auch Silylesterschutzgruppen, insbesondere die Trimethylsilylschutzgruppe, die z.B. durch Reaktion der freien Säure mit N,O-Bis-(trimethylsilyl)acetamid eingeführt werden kann.

Die 7-Aminogruppe des Ausgangsmaterials der Formel V kann ebenfalls geschützt sein. Geeignete Schutzgruppen sind bekannt und umfassen z.B. die Trimethylsilylgruppe, die beispielsweise gleichzeitig mit Schützung der Carboxylgruppe eingeführt werden kann.

Der Aminosubstituent im Ausgangsmaterial IV/IVa kann in freier oder geschützter Form vorliegen. Wie bereits ausgeführt, ist im allgemeinen ein Schutz nicht notwendig. Sollte jedoch trotzdem ein Schutz erwünscht sein, so kann dies in bekannter Weise, unter Verwendung geeigneter bekannter Schutzgruppen, durchgeführt werden.

Nach der Reaktion der Verbindungen der Formel IV und V können nachfolgende Entschützungsreaktionen in bekannter Weise durchgeführt werden. Ebenso kann die Überführung der freien Säureform ($R_3$ = COOH) in die Salze nach an sich bekannten Methoden ausgeführt werden.

Die Endprodukte können nach bekannten Methoden isoliert und gereinigt werden.

Im erfindungsgemäßen Verfahren verwendet man als reaktives Derivat der Säure der Formel A heterocyclische Thioester. Es wurde überraschenderweise gefunden, daß diese Ester hergestellt und verwendet werden können. Weiters wurde überraschenderweise gefunden, daß eine im Heterocyclus dieser Ester vorhandene Aminogruppe nicht zur Selbstreaktion führt. Demzufolge ist ein Schützen dieser Aminogruppe in der folgenden Acylie rung nicht notwendig. Andererseits kann die Aminogruppe selbstverständlich geschützt werden, falls dies aus einem anderen Grund gewünscht wird.

Bei den Verbindungen der Formel IV ist die Art des Ringes

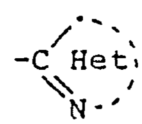

bzw.

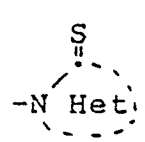

nicht kritisch, die bevorzugten Verbindungen werden durch Faktoren wie Leichtigkeit bei der Herstellung und Verfügbarkeit des Ausgangsmaterials bestimmt. Vorzugsweise bedeutet der Ring 2-Pyridyl oder insbesondere 2-Benzothiazolyl. Er kann auch für Pyrimidinyl, Triazolyl oder Thiazolyl stehen.

Die Verbindungen der Formel IV können durch Veresterung von Verbindungen der Formel

$R_1$ - COOH    VI

worin $R_1$ obige Bedeutung besitzt, erhalten werden.

Die Veresterung kann beispielsweise durch Reaktion mit einer Verbindung der Formel

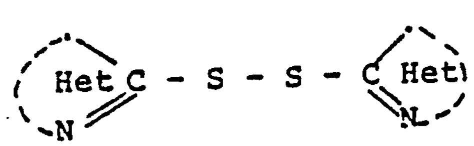

VII

worin beide

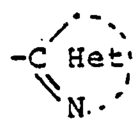

gleich sind und obige Bedeutung besitzen, durchgeführt werden.

Diese Veresterung wird vorzugweise unter Gegenwart eines Tri(niederalkyl)oder Tri(aryl)phosphins oder -phosphits, insbesondere Triphenylphosphins, durchgeführt, und zwar vorzugsweise bei einer Temperatur von -30 bis +50° C, insbesondere bei -20 bis +25° C und vorteilhafterweise bei -5 bis +5° C. Als Lösungsmittel wird ein inertes, nicht hydroxylgruppenhältiges organisches Lösungsmittel, z.B. ein chlorierter Kohlenwasserstoff, wie Dichlormethan, eingesetzt. Wird eine Verbindung der Formel IV gewünscht, worin in $R_1$ die Aminogruppe geschützt ist, so kann die Aminoschutzgruppe vor oder nach der Veresterung eingeführt werden. Die Endprodukte können durch Behandeln mit niederen Alkoholen kristallisiert und gereinigt werden, wobei darauf geachtet werden muß, daß die Temperatur 20° C, vorzugsweise 0° C, nicht übersteigt.

Die Verbindungen der Formel I sind, wie bereits erwähnt, Zwischenprodukte zur Herstellung bekannter Antibiotika. Diese entfalten eine Hemmwirkung gegen Bakterien, wie sich durch Untersuchungen in vitro mit dem Reihenverdünnungstest und in vivo durch Versuche an Mäusen, unter Verwendung verschiedener Stämme, z.B. von Staphylococcus aureus, Streptococcus pyogenes, Streptococcus faecalis, Escherichia coli, Proteus vulgaris, Proteus mirabilis, Proteus morganii, Shigella dysenteria, Shigella sonnei, Shigella flexneri, Alcaligenes faecalis, Klebsiella aerogenes, Klebsiella pneunomiae, Serrata marcescens, Salmonella Heidelberg, Salmonella typhimurium, Salmonella enteritidis und Neisseria gonorrhoae, zeigen läßt. Diese Hemmwirkung wurde ab einer Konzentration von ca. 0,01 bis 50 µg/ml bzw. ab einer Dosis von ca. 0,1 bis 100 mg/kg Körpergewicht festgestellt.

Daher können diese Cephalosporinantibiotika als antibakteriell wirksame Antibiotika verwendet werden. Für diese Verwendung hängt die zu verabreichende Dosis von der verwendeten Verbindung und der Verabreichungsart sowie der Behandlungsart ab. Man erhält zufriedenstellende Ergebnisse bei Verabreichung einer täglichen Dosis von ca. 1 bis 6 g. Diese Menge kann gegebenenfalls in entsprechend kleineren Dosen von 0,25 bis 3 g zwei- bis viermal täglich oder in Retardform gegeben werden.

Die Verbindungen, worin $R_3$ für Carboxyl steht, können in Form der freien Säuren oder ihrer physiologisch verträglichen Salze eingesetzt werden, wobei die Salze größenordnungsmäßig die gleiche Wirksamkeit besitzen wie die freien Säuren. Geeignete Salzformen sind beispielsweise Alkali- und Erdalkalimetallsalze; vorzugsweise Alkalimetallsalze, insbesondere Natriumsalze. Die Verbindungen können gemeinsam mit anorganischen oder organischen pharmakologisch indifferenten Verdünnungs- oder Trägerstoffen bzw. Hilfsstoffen verabreicht werden. Beispielsweise werden sie als Bestandteil von Kapseln, Injektions- oder Instillationszubereitungen eingesetzt.

In den nachfolgenden Beispielen, die die Erfindung näher erläutern, ihren Umfang aber in keiner Weise einschränken sollen, erfolgen alle Temperaturangaben in Celsiusgraden.

Beispiel 1: 7-[[(2-Amino-4-thiazolyl)-2-oxoacetyl]amino]-3-[[(1-methyl-1H-tetrazol-5-yl)thio]methyl]-8-oxo-5-thia-1-azabicyclo[4.2.0]oct-2-en-2-carbonsäure:

3,29 g 7-Amino-3-[(1-methyl-1H-tetrazol-5-yl)thiomethyl]ceph-3-em-4-carbonsäure werden in 25 ml Dichlormethan suspendiert und mit 2,7 ml N,O-Bis-(trimethylsilyl)acetamid versetzt. Nach 15 Minuten liegt eine klare Lösung vor. Man kühlt auf -15° und gibt 3,9 g 2-(2-Aminothiazol-4-yl)-2-oxothioessigsäure-S-benzothiazol-2-ylester zu. Nach vierstündigem Rühren bei -15° wird der Ansatz in 100 ml Ethanol eingerührt, wobei das Endprodukt ausfällt. Man rührt eine Stunde bei 0°, filtriert, wäscht mit Ethanol und trocknet im Vakuum. Ausbeute: 3,9 g, d.i. 80,7 %. Fp: ab 145° (Zers.).
IR: 3350 N-H, 1800 C=O (ß-Lactam), 1690 C=O (Amid), 1545 (Amid II).

Beispiel 2: 7-[[(2-Amino-4-thiazolyl)-2-oxoacetyl]amino]-3-(1-pyridinium)methyl-8-oxo-5-thia-1-azabicyclo-[4.2.0]oct-2-en-carbonsäure.Chlorid:

3,28 g 7-Amino-3-(1-pyridiniummethyl)ceph-3-em-4-carbonsäure.Chlorid werden in 25 ml Dichlormethan suspendiert und mit 3 ml N,O-Bis-(trimethylsilyi)acetamid versetzt. Nach 10 Minuten liegt eine klare Lösung vor. Man kühlt auf -15° und gibt 3,9 g 2-(2-Aminothiazol-4-yl)-2-oxothioessigsäure-S-benzothiazol-2-ylester

zu. Nach vierstündigem Rühren bei -15° wird der Ansatz in 100 ml Ethanol eingerührt, wobei das Endprodukt ausfällt. Man rührt eine Stunde bei 0°, filtriert, wäscht mit Ethanol und trocknet im Vakuum. Ausbeute: 4,0 g, d.i. 83,0 %. Fp: ab 162° (Zers.).
IR: NH 3300 (breit), 1765, C=O (ß-Lactam), 1645, C=O (Amid), 1610, (Amid II).

Beispiel 3: 7-[[(2-Amino-4-thiazolyl)-2-oxoacetyl]amino]-3-acetoxymethylceph-3-em-4-carbonsäure:

5,44 g 7-Amino-3-acetoxymethylceph-3-em-4-carbonsäure werden unter Stickstoffatmosphäre in 50 ml trockenem Dichlormethan suspendiert und mit 5,4 ml N,O-Bis(trimethylsilyl)acetamid versetzt. Der Ansatz wird bei Raumtemperatur gerührt, bis eine klare Lösung vorliegt, und dann auf -15° gekühlt. Man gibt 8,4 g 2-(2-Amino-4-thiazolyl)-2-oxothioessigsäure-S-benzthiazol-2-ylester zu und rührt zwei Stunden bei -15° und dann zwei Stunden bei 0°. Das Lösungsmittel wird am Rotavapor schonend abgezogen und der ölige Rückstand mit 50 ml Ethanol verrieben, wobei die Titelverbindung ausfällt. Man erhält 6,3 g in Form eines gelben Pulvers.
IR: 3335 NH, 1800 c = o (ß-Lactam), 1755 c = o, 1685 c = o (Amid), 1515 (Amid II)

Beispiel 4: 7-[[(2-Amino-4-thiazolyl)-2-oxoacetyl]amino]-3-methylceph-3-em-4-carbonsäure:

4,28 g 7-Amino-3-methylceph-3-em-4-carbonsäure werden unter Stickstoffatmosphäre in 80 ml trockenem Dichlormethan suspendiert und mit 5,4 ml. N,O-Bis(trimethylsilyl)acetamid versetzt. Der Ansatz wird bei Raumtemperatur gerührt, bis eine klare Lösung vorliegt, und dann auf -15° gekühlt. Man gibt 8,4 g 2-(2-Amino-4-thiazolyl)-2-oxothioessigsäure-S-benzthiazol-2-ylester zu und rührt eine Stunde bei -15°, eine Stunde bei 0° und dann zwei Stunden bei +20°. Das Lösungsmittel wird am Rotavapor schonend abgezogen und der ölige Rückstand mit 50 ml Ethanol verrieben. Der entstandene Niederschlag wird filtriert, mit Ethanol und Ether gewaschen und im Vakuum bei 40° getrocknet. Man erhält 5,3 g der Titelverbindung als gelbes Pulver.
IR: 3340 NH, 1790 c = o (ß-Lactam),1685 c = o, 1540 (Amid II).

Beispiel 5: 7-[[(2-Amino-4-thiazolyl)-2-oxoacetyl]amino]-3-(1,2,3-triazol-5-ylthiomethyl)ceph-3-em-4-carbonsäure:

6,26g 7-Amino-3-(1,2,3-triazol-5-ylthiomethyl)ceph-3-em-4-carbonsäure werden in 50 ml Dichlormethan durch Zugabe von 5,4 ml N,O-Bis(trimethylsilyl)acetamid in den Silylester überführt. Man kühlt auf -15° und gibt 10,1 g 2-(2-Amino-4-thiazolyl)-2-oxothioessigsäure-S-benzthiazol-2-ylester in Form des Tetrahydrofuransolvates zu. Nach zwei Stunden bei -15° und einer Stunde bei 0° wird das Dichlormethan am Rotavapor entfernt und der Rückstand mit 100 ml Methanol verrührt. Der Niederschlag wird in bekannter Weise isoliert. Man erhält 8,4 g der Titelverbindung als gelbes Pulver.
IR: 3320 NH (breit), 1790 c = o (ß-Lactam, 1680 c = o, 1530 (Amid II).

Beispiel 6: 7-[[(2-Amino-4-thiazolyl)-2-oxoacetyl]amino]-3-[(2,3-cyclopenteno-1-pyridinium)methyl]ceph-3-em-4-carboxylat

9,88 g 7-Amino-3-[(2,3-cyclopenteno-1-pyridinium)methyl]ceph-3-em-4-carboxylat.Hydrojodid.Monohydrat werden in 100 ml Dichlormethan mit 11,4 ml N,O-Bis(trimethylsilyl)acetamid silyliert. Die entstandene Lösung wird auf -15° gekühlt und mit 8,4 g 2-(2-Amino-4-thiazolyl)-2-oxothioessigsäure-S-benzthiazol-2-ylester versetzt. Man rührt drei Stunden bei -15° und setzt dann 100 ml Ethanol und 3 ml Triethylamin zu. Der Ansatz wird eine Stunde bei 0° gerührt, der entstandene Niederschlag abfiltriert, gewaschen und getrocknet. Man erhält 11,5 g der Titelverbindung als oranges Pulver.
IR: 3300 NH, 2965 $CH_2$, 1795 c = o (ß-Lactam), 1690 c = o, 1635 (Amid I), 1545 (Amid II).

Beispiel 7: 7-[[(2-Amino-4-thiazolyl)-2-oxoacetyl]amino]-3-[(2,5-dihydro-6-hydroxy-2-methyl-5-oxo-1,2,4-triazin-3-yl)thiomethyl]ceph-3-em-4-carbonsäure:

7,4 g 7-Amino-3-[(2,5-dihydro-6-hydroxy-2-methyl-5-oxo-1,2,4-triazin-3-yl)thiomethyl]ceph-3-em-4-carbonsäure (Wassergehalt: 3,7 %) werden in 50 ml Dichlormethan bei 30° mit 14,85 ml N,O-Bis-(trimethylsilyl)acetamid versetzt. Nach zwei Stunden bei 30° wird auf -15° gekühlt. Man gibt 8,1 g 2-(2-Amino-4-thiazolyl)-2-oxothioessigsäure-S-benzthiazol-2-ylester zu und rührt sechs Stunden bei -15°. Der Ansatz wird zu 500 ml auf 50° erwärmtes Ethanol gegeben, unter Rühren abgekühlt und eine Stunde bei

0° gerührt. Der Niederschlag wird abfiltriert, gewaschen und getrocknet. Man erhält 9,1 g der Titelverbindung als gelbes Pulver.
IR: 3320 NH, 1805 c = o, 1735 c = o, 1690 c = o, 1645 (Amid I), 1555 (Amid II).

HERSTELLUNG DER AUSGANGSPRODUKTE IV

Beispiel 8: 2-(2-Aminothiazol-4-yl)-2-oxothioessigsäure-S-benzothiazol-2-ylester:

34,4 g Aminothiazolylglyoxylsäure werden in 500 ml Dichlormethan suspendiert und unter Rühren mit 28 ml Triethylamin versetzt. Man kühlt auf -15° und gibt 64 g Triphenylphosphin und 80 g Bisbenzothiazol-2-yldisulfid zu. Nach zweistündigem Rühren bei - 15° wird der orange Niederschlag abfiltriert, mit kaltem Dichlormethan gewaschen und im Vakuum getrocknet. Ausbeute: 57,7 g, d.i. 89,8 %.
IR: 3300 N-H, 1675 Carbonyl (Thioester), 1645 Carbonyl (Amid), 1540 Amidbande.

Beispiel 9: 2-(2-Aminothiazol-4-yl)-2-oxothioessigsäure-S-benzothiazol-2-ylester:

80 g Bisbenzothiazol-2-yldisulfid und 64 g Triphenylphosphin werden in 500 ml Tetrahydrofuran suspendiert und auf -15° gekühlt. Man gibt 34,4 g Aminothiazolylglyoxylsäure zu und tropft 16,1 ml Pyridin so langsam zu, daß die Innentemperatur nicht über -15° ansteigt. Die orange Suspension wird zwei Stunden bei -15° gerührt. Der Niederschlag wird abfiltriert, mit kaltem Tetrahydrofuran gewaschen und im Vakuum getrocknet. Ausbeute: 62,2 g als Tetrahydrofuran-Solvat. Fp: bei 80° Entweichen von THF, ab 123° (Zers.). THF-Gehalt (gaschromatographisch): 17 %

**Patentansprüche**

**1.** Verfahren zur Herstellung von Cephalosporinzwischenprodukten der Formel

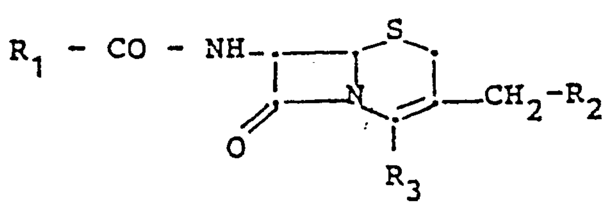

I

worin $R_1$ $R_1$ für die Gruppe der Formel

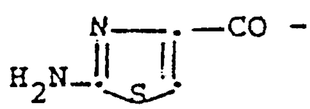

IIa

steht, $R_2$ für Wasserstoff, die Acetoxy-, die Carbamoyloxy-, eine S-Y-Gruppe, wobei Y einen unsubstituierten oder substituierten Hetero cyclus bedeutet, oder $R_2$ für ein gegebenenfalls substituiertes Pyridinium der Formel

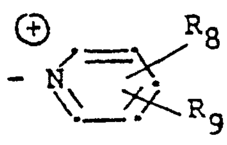

III

wobei $R_8$ und $R_9$ gleich oder verschieden sind und jeweils für Wasserstoff, Halogen, Alkyl, Hydroxy, Carboxamido, Alkoxycarbonyl, Amino, Monoalkylamino oder Dialkylamino stehen oder zusammen einen gegebenenfalls substituierten, 5- oder 6-gliedrigen carbocyclischen Ring bedeuten, steht und $R_3$ die Carboxyl-, die Carboxylat- oder eine Carbonsäureestergruppe bedeutet, dadurch gekennzeichnet, dass man eine Verbindung der Formel

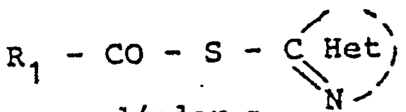 IV

und/oder

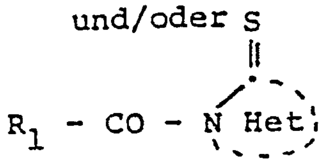 IVa.

worin $R_1$ obige Bedeutung besitzt, und

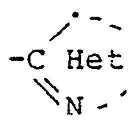 bzw. 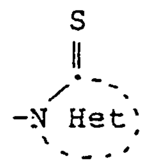

für einen 5- oder 6-gliedrigen Heterocyclus, der zusätzlich zum N-Atom ein oder zwei weitere Heteroatome beinhalten kann, beispielsweise O, N und S, und der substituiert oder mit einem gegebenenfalls substituierten Benzolring annelliert sein kann, stehen, mit einer Verbindung der Formel

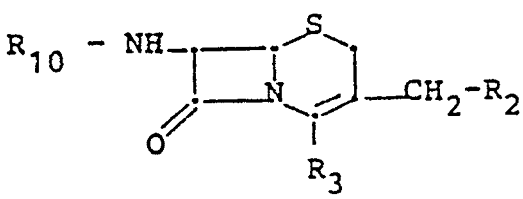 V

worin $R_2$ und $R_3$ obige Bedeutung besitzen und $R_{10}$ für Wasserstoff oder eine Aminoschutzgruppe steht, umsetzt und gewünschtenfalls ein erhaltenes Endprodukt entschützt und gegebenenfalls ein erhaltenes Endprodukt, worin $R_3$ für COOH steht, in ein Salz überführt oder umgekehrt.

2. Verfahren nach Ansprüch 1, worin in den Verbindungen der Formel IV

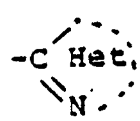

für 2-Pyridyl steht.

3. Verfahren nach Ansprüch 1, worin in den Verbindungen der Formel IV

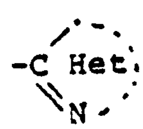

für 2-Benzthiazolyl steht.

## Claims

1. Process for the preparation of cephalosporine intermediate products of formula

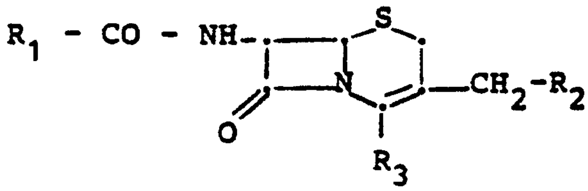

I

wherein $R_1$ denotes the group of formula

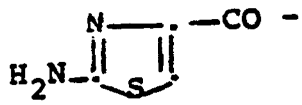

IIa

$R_2$ denotes hydrogen, the acetoxy, the carbamoyloxy, a S-Y- group, whereby Y signifies an unsubstituted or substituted heterocycle, or $R_2$ denotes an optionally substituted pyridinium of formula

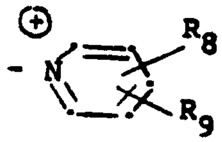

III

whereby $R_6$ and $R_9$ are the same or different and each denotes hydrogen, halogen, alkyl, hydroxy, carboxamido, alkoxycarbonyl, amino, monoalkylamino or dialkylamino, or together signify an optionally substituted, 5- or 6-membered, carbocyclic ring, and $R_3$ signifies the carboxyl, the carboxylate or a carboxylic acid ester group, characterised by reacting a compound of formula

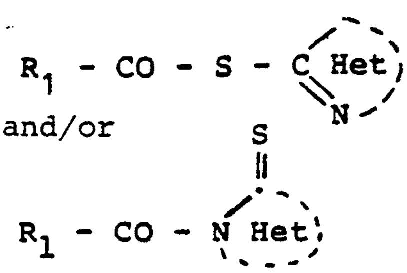

IV

and/or

IVa

wherein $R_1$ is defined as above, and

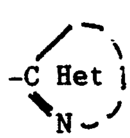

resp.

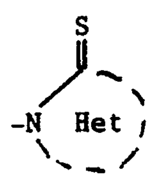

denote a 5- or 6-membered heterocycle, which may contain in addition to the N-atom one or two further heteroatoms, for example O, N and S, and which may be substituted or anellated with an optionally substituted benzene ring, with a compound of formula

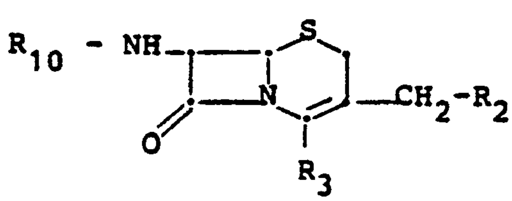

V

wherein $R_2$ and $R_3$ are defined as above, and $R_{10}$ denotes hydrogen or an amino protecting group, and if desired, deprotecting a final product obtained and optionally converting a final product obtained, in which $R_3$ denotes COOH, into a salt, or vice versa.

2. Process according to claim 1, wherein in the compounds of formula IV,

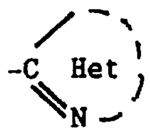

denotes 2-pyridyl.

3. Process according to claim 1, wherein in the compounds of formula IV,

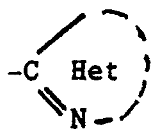

denotes 2-benzothiazolyl.

**Revendications**

1. Procédé de préparation de produits intermédiaires céphalosporiniques de formule

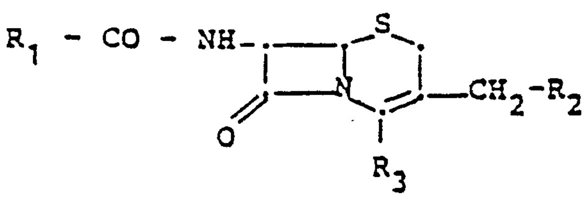

I

dans laquelle

$R_1$ représente le groupe de formule

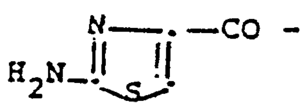

IIa

$R_2$ représente l'hydrogène, le groupe acétoxy, le groupe carbamoyloxy, un groupe S-Y où Y signifie un hétérocycle non substitué ou substitué, ou bien $R_2$ représente un pyridinium éventuellement substitué de formule

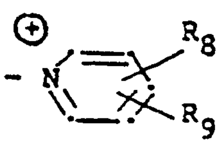

III

où $R_8$ et $R_9$ sont identiques ou différents et représentent chacun l'hydrogène, un halogène ou un groupe alkyle, hydroxy, carboxamido, alcoxycarbonyle, amino, monoalkylamino ou dialkylamino, ou signifient ensemble un carbocycle à 5 ou 6 chaînons éventuellement substitué, et $R_3$ signifie un groupe carboxy, carboxylate ou ester d'acide carboxylique, caractérisé en ce

qu'on fait réagir un composé de formule

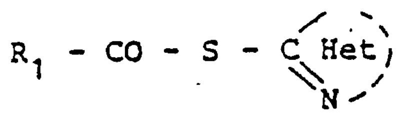 IV

et/ou

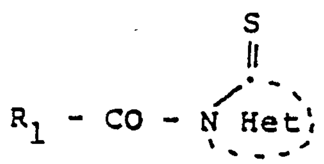 IVa

dans lesquelles $R_1$ a la signification donnée ci-dessus et respectivement

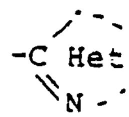

et

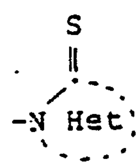

représentent un hétérocycle à 5 ou 6 chaînons qui peut contenir, en plus de l'atome d'azote, un ou deux autres hétéroatomes, par exemple O, N et S, et qui peut être substitué ou qui peut être condensé avec un cycle benzénique éventuellement substitué, avec un composé de formule

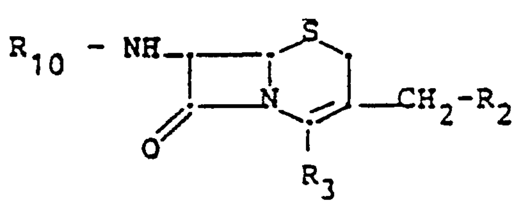 V

dans laquelle $R_2$ et $R_3$ ont les significations données ci-dessus et $R_{10}$ représente l'hydrogène ou un groupe protecteur du groupe amino, et éventuellement on déprotège un produit final obtenu et éventuellement on transforme un produit final obtenu, dans lequel $R_3$ représente COOH, en un sel, ou vice versa.

2. Procédé selon la revendication 1, caractérisé en ce que, dans les composés de formule IV, le groupe de formule

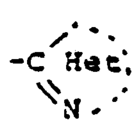

représente un groupe 2-pyridyle.

3. Procédé selon la revendication 1, caractérisé en ce que, dans les composés de formule IV, le groupe de formule

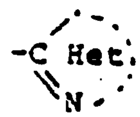

représente un groupe 2-benzothiazolyle.